# EUROPEAN PATENT APPLICATION

(11) **EP 4 670 636 A1**
(43) Date of publication of application: **31.12.2025**
(21) Application number: 24184545.2
(22) Date of filing: 26.06.2024
(51) Int. Cl.: A61B 6/04, A61B 6/58, A61B 6/50

(54) **MOTION PHANTOM**

(71) Applicant: Koninklijke Philips N.V., 5656 AG Eindhoven (NL)
(72) Inventor: FRERKING, Lena Christina, Eindhoven (NL); SENEGAS, Julien Thomas, Eindhoven (NL); WEISS, Steffen, 5656AG Eindhoven (NL); LOESCHER, Stefan Leonhard, Eindhoven (NL)
(74) Representative: Philips Intellectual Property & Standards

(57) **Abstract**

There is described an apparatus (200) for use with a medical imaging system (10). The apparatus comprises a phantom object (202) to be imaged, a linear actuator (206), an imaging stage (204) configured to receive the phantom object, and coupling means (214) configured to couple the imaging stage to the linear actuator. The coupling means are configured to generate at least one of a tilting motion (214a), a rotating motion (214b), and a lifting motion (214c), of the phantom object, when the linear actuator is in operation.

## Description

### FIELD OF THE INVENTION

The subject-matter of the present disclosure relates to motion phantoms for design, test, SW-development, and calibration of medical imaging systems and workflows. In particular, the present disclosure is concerned with a chest phantom for simulating human chest motion, and an apparatus to generate motion in a phantom object.

### BACKGROUND OF THE INVENTION

When testing different settings for medical image devices, such as computed tomography (CT) or magnetic resonance (MR) devices, it is common to use phantoms that offer realistic reconstructed images regarding certain aspects of the human body when being imaged. Such phantoms might include abstract shapes, or parts resembling that of a body, and be made from materials which provide similar HU values and/or T1/T2 relaxation times to a particular body part. Some phantoms may also involve moving parts, with the displacement of the phantom parts causing artifacts in the reconstructed image, the analysis of which may serve as basis for refinement of the imaging system.

Existing motion phantoms, however, can realize motion in one or maximally two distinct directions. They typically mimic rigid body motion, without any deformation of the moving parts. Actual human physiological motion is, however, usually much more complex. E.g., while breathing, organs are shifted in different directions. Certain parts of the body move in anterior-posterior (AP) direction, others in left-right direction, again other parts in feet-head direction, and so on. Hence, a realistic motion is not rigid-body-like, but rather elastic, which leads to more complex kinds of artifacts than those that can be realized with state-of-the-art phantoms; rather, state of the art phantoms typically mimic one body part with a particular motion, so that a number of different phantoms must be built or acquired if several types of motions need to be analyzed.

Moreover, the requirements for phantoms serving for CT or MR experiments are quite different than for those serving for camera experiments. If the effect of a camera-detected motion event for a reconstructed CT or MR image shall be analyzed, the information of camera and medical imaging device needs to be combined in a single experiment. However, CT specific motion phantoms do not have a human-like appearance, whereas mannequin-like motion phantoms that look realistic to a camera, do not have appropriate interior materials for analyzing CT or MR scans.

It is therefore an aim of the present disclosure to improve on the prior art.

### SUMMARY OF THE INVENTION

The present invention is defined according to the claims.

According to a first aspect of the present invention, there is provided a phantom simulating human chest motion for calibrating a medical imaging system. The phantom comprises a first flexible fluid container hydraulically coupled to a second flexible fluid container which are caused to sequentially inflate and deflate by a pumping means hydraulically coupled to the first and second flexible fluid containers. The phantom comprises a set of tissue simulacrum for mimicking certain human tissues in a medical image, and a ribcage-like structure within which the first and second flexible fluid containers are arranged. In an example, the first and second flexible fluid containers may be filled with an inert gas, such as air.

In an example, a first simulacrum in the set of tissue simulacrum may comprise one or more sponges soaked in a first aqueous fluid. A second simulacrum in the set of tissue simulacrum may comprise one or more fluid conduits comprising a second aqueous fluid. The first and second simulacra may be disposed inside at least one of the first and second flexible fluid containers, and furthermore may be attached to a wall of the respective container. A third simulacrum in the set of tissue simulacrum may comprise one or more substantially solid blocks. A fourth simulacrum in the set of tissue simulacrum may comprise one or more fluid bags comprising a third aqueous fluid. The fourth simulacrum may be positioned between the flexible fluid containers and the ribcage-like structure. A fifth simulacrum in the set of tissue simulacrum may comprise one or more fluid bags comprising a fourth aqueous fluid. The fifth may be disposed surrounding the ribcage-like structure. Each of the first to fourth aqueous fluids may be different.

In an example, the phantom may comprise a resilient coupling configured to return the first and second flexible fluid containers to a default position during deflation.

In an example, the ribcage-like structure may comprise a plurality of separate ribs and a sternum. The plurality of ribs may be attached to the sternum by a synthetic polymer thread, such as nylon, which is invisible to the medical imaging.

In an example, at least one opposing pair of ribs may comprise biasing means configured to bias the pair of ribs towards each other, and so aid with exhalation.

In an example, the pumping means comprises a third flexible fluid container hydraulically coupled to the first and second flexible fluid containers, and means to apply pressure to the third flexible fluid container.

According to a second aspect of the invention, there is provided a medical imaging system comprising an image sensor and the aforementioned phantom, wherein the image sensor is configured to capture a plurality of images of the phantom while it is being caused to inflate and deflate.

In an example, the medical imaging system may be a magnetic resonance imaging system. In another example, the medical imaging system may be a computed tomography scanner.

According to a third aspect of the invention, there is provided an apparatus for calibrating (or otherwise testing or aiding with design of) a medical imaging system. The apparatus comprises a phantom object to be imaged, an imaging stage configured to receive the phantom object, a linear actuator, and coupling means configured to couple the imaging stage to the linear actuator. The coupling means are configured to generate at least one of a tilting motion, a rotating motion, and a lifting motion, of the phantom object, when the linear actuator is in operation.

In an example, the coupling means may be a first coupling means in a set of coupling means, and the set may comprise at least a second coupling means configured to generate at least one of a tilting motion, a rotating motion, and a lifting motion, of the phantom object, different to the motion of the first coupling means. The linear actuator may be configured to be interchangeable between the first coupling means and second coupling means.

In an example, coupling means configured to generate titling motion may comprise a member with a first end coupled to the linear actuator and a second end fixedly attached to the imaging stage.

In an example, coupling means configured to generate rotating motion may comprise a member with a first end coupled to the linear actuator and a second end coupled to a first drive shaft of a gear system, the gear system comprising a second draft shaft oriented at an angle to the first drive shaft. The gear system may comprise a beveled gear arrangement or a worm gear arrangement.

In an example, coupling means configured to generate lifting motion may comprise a pair of abutting wedges having a first wedge coupled to the linear actuator and a second wedge coupled to the imaging stage, and wherein pushing and pulling the first wedge using the linear actuator causes the second wedge to rise and fall, respectively.

In an example, the imaging stage may comprise one or more counterweights configured to support the at least one tilting motion, rotating motion, and a lifting motion, particularly where the phantom object is a head phantom.

In an example, the apparatus may comprise a power unit configured to drive the linear actuator, such as a stepper motor.

In an example, the apparatus may comprise an imaging sensor arranged to capture images of the phantom object in motion, and a controller configured to transmit a trigger signal to the image sensor to capture an image based on the operation of the power unit.

The phantom object may be one of a chest phantom (as above), a head phantom, a hand phantom, or any other suitable phantom of a human (or animal) body. In an example, the phantom object may comprise a non-metallic skin-like mask, and/or may be shrouded in clothing-like material.

These and other aspects of the present invention will be apparent from and elucidated with reference to the embodiment(s) described hereinafter.

### BRIEF DESCRIPTION OF DRAWINGS

The embodiments of the present inventions may be best understood with reference to the accompanying Figures, in which:
Fig. 1 shows an example medical imaging system;
Fig. 2 shows one view of an example chest phantom; and
Fig. 3 shoes another view of an example chest phantom;
Fig. 4 shows an example motion phantom apparatus;
Fig. 5 shows another example motion phantom apparatus.

### DESCRIPTION OF EMBODIMENTS

Nowadays, CT and MR scanners are often equipped with cameras, which are, e.g., attached to the gantry or at the ceiling above the patient couch. Also, for experimenting with certain characteristics of the camera images or the associated software, phantoms can be used. While for a CT/MR scan it is rather important that the interior behavior of a phantom is realistic, for the camera the outer appearance is crucial. If the camera software is supposed to recognize specific parts of the body, then a phantom should have a realistic shape and (optionally) color. If the software shall detect motion, then the motion of the phantom should mimic the human motion as realistically as possible.

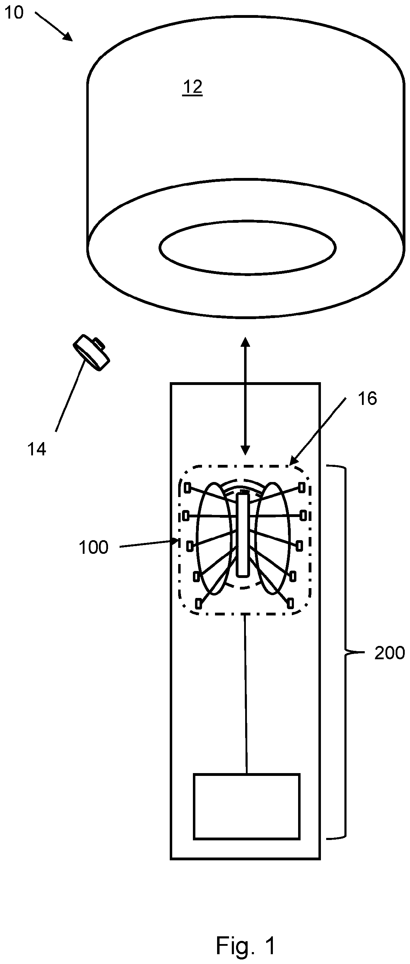

Fig. 1 shows an example medical imaging system 10. The medical imaging system 10 comprises a suitable imaging apparatus 12 such as an MR or a CT scanner. The system also comprises an imaging sensor 14 configured to capture real-time images (of visible light) of a target 16 being imaged by the apparatus 12. That is, the imaging apparatus 12 and imaging sensor 14 may be configured to capture images of the target 16 while it is undergoing motion.

The target 16 may be a patient or an object. An example object may be a phantom object which is configured to imitate a particular part of a body that might be expected to move (inadvertently) during a medical scan. For example, the target 16 may be a phantom 100 as discussed in detail below. The target 16 may be part of a motion phantom, such as apparatus 200 discussed below, configured to generate movement in the target 16 while it is being scanned by the medical imaging system 10.

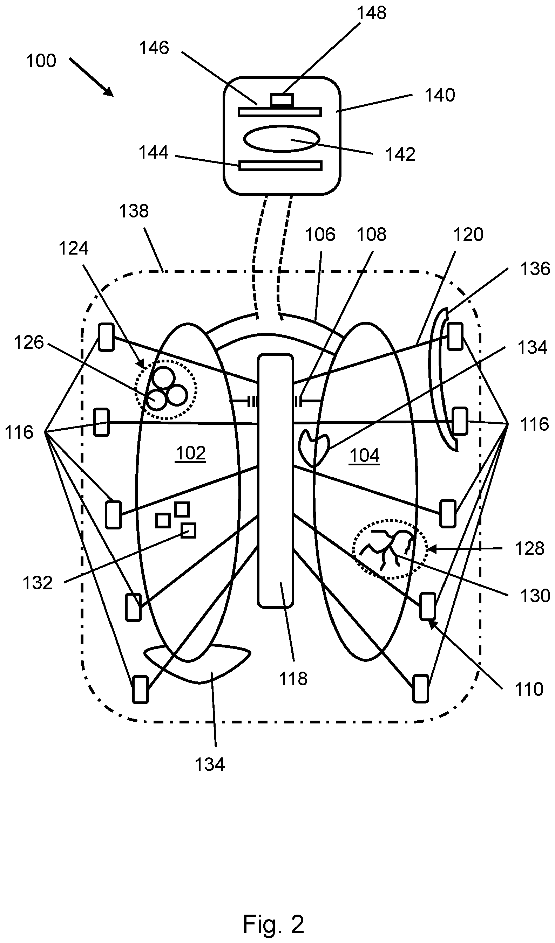

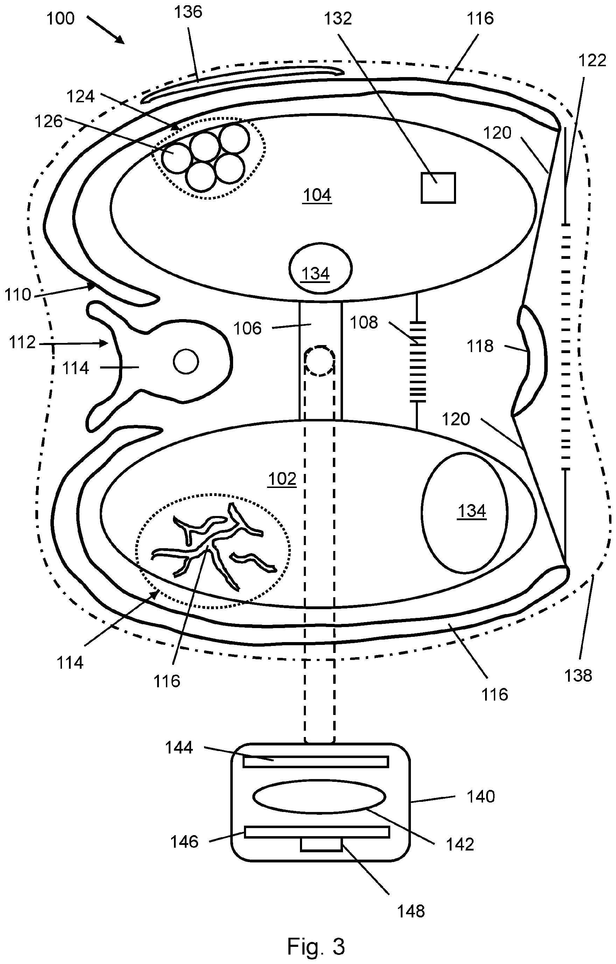

With reference to Figs 2 and 3, there is shown an example phantom 100 designed to mimic realistic breathing motion. The phantom 100 may also be termed a chest phantom, as this is the part of the human body that it is designed to mimic. The phantom 100 is suitably designed for use in a medical imaging system, such as CT or MR imaging, in order to aid in design, testing, software development, and calibration of the imaging systems and workflows.

The phantom 100 comprises a pair of flexible fluid containers - that is, a first fluid container 102 and a second fluid container 104 - which are hydraulicly coupled by suitable fluidic coupling means such as piping 106. The two fluid containers 102, 104 may be elongate in shape, having length greater than width, are separated laterally, and aligned parallel with each other. In this way the fluid containers 102, 104 are disposed to mimic the left and right lungs of a body. Suitably, the containers 102, 104 may be formed from a material having a Hounsfield unit similar to lung tissue, for example in the range -700 to -600 HU.

The two containers 102, 104 are hermetically sealed apart from an opening in each container 102, 104 which allows for connection to the piping 106. The piping 106 is configured to provide fluid flow into, and out of, the containers 102, 104. Suitably, it will be appreciated that the pair of containers 102, 104 are inflatable and collapsible, such that cycling through inflation and deflation periods mimics lung expansion from breathing. Beneficially, the fluid containers are able to expand in all possible directions. In order to ensure that containers 102, 104 return to a suitable (default) rest position after inflation/during deflation, then phantom 100 may comprise a resilient coupling 108, such as a spring, configured to return the pair of containers 102, 104 to their default rest position. It will be appreciated from the above that the containers 102, 104 and piping 106 may be configured to contain a suitable fluid for phantom imaging: standard air is of course most appropriate, but in general any inert gas may be used.

The phantom 100 comprises a ribcage-like structure 110, within which the first and second flexible fluid containers 102, 104 are arranged. That is, much like a real chest which the phantom aims to mimic, the first and second fluid containers 102, 104 are housed within the rib cage 110.

The rib cage like structure 110 is formed to mimic bone, and therefore may be formed from a material having a Hounsfield unit value between about 300 HU and 1900 HU. In particular, the ribcage 110 may be formed from two different materials, a first material representing cancellous bone and a second material representing cortical bone; the first material may have HU between about 300 and 500, and the second material HU between about 500 and 1900. In an example, the ribcage 110 may be formed from a single material having HU about 1000.

Suitably, the ribcage-like structure 110 comprises a spine 112 having a number of vertebrae 114 corresponding to a plurality of separate ribs 116, a sternum 118. The plurality of ribs 116 may be attached to the sternum 118 by a synthetic polymer thread 120, such as nylon. In this way the ribs may be generally held in position but be provided with some scope to move in response to inflation of the containers 102, 104. Beneficially, the synthetic thread should not show up as an artefact on a medical scan.

Moreover, by adjusting the angle between the ribs 116 and the vertebrae 114, typically between 30 to 60 degree like for the human rib cage, the motion of the ribs 116 can have components along the anterior-posterior, left-right and feet-head directions. In an example, the tips of a left-right pair of ribs may be connected by biasing means 122, such as a rubber band, arranged to bias the tips of the pair of ribs towards each other in order to provide a restoring force to return the ribs to a default (rest) position during an exhale of the containers 102, 104.

In order to mimic various tissue structures of the chest (in and around the lungs), the phantom 100 suitably comprises a set of tissue simulacrum arranged in and around the first and second containers 102, 104. Suitably, the set of tissue simulacrum comprises objects which are intended to mimic different types of tissue that one expects to detect when capturing medical images of a chest (e.g., using an imaging apparatus 202 as in Fig. 1).

A first simulacrum 124 in the set of tissue simulacrum comprises one or more sponges 126 soaked in a first aqueous fluid. The combination of sponge and aqueous fluid may be selected in order to mimic inner structures of a lung that one might expect to see on the appropriate medical imaging device. For example, where the phantom 100 is being designed for use with a CT scanner, the first simulacrum may be configured to have a Hounsfield unit between -700 and -600 (although not necessarily the same value as the containers 102, 104). The first simulacrum 124 (or simulacra, when more than one sponge is used) may be disposed inside one or both of the containers 102, 104, and may be attached to the inside wall of the respective container(s) so that the first simulacrum 124 moves with the inflation of the containers 102, 104, thereby creating motion artefacts in a medical image.

A second simulacrum 128 in the set may comprise one or more fluid conduits 130 comprising a second aqueous fluid, which may be provided to mimic vessel structures within the lungs such as blood vessels. Suitably, the second aqueous fluid may be different to the first aqueous fluid so that the second simulacrum 128 appears different to the first simulacrum in the medical imaging. In particular, the second fluid may be provided to approximate blood in a medical image. For example, for CT scanning the second fluid may have a Hounsfield unit from about +13 to +75. In another example, for MR imaging, the second fluid may have a T1 of 1500ms and T2 of 280ms, being formed from tap water with a low concentration of Gadolinium based contrast agent.

Second simulacra may 128 may be disposed within one or both of the first and second fluid containers 102, 104. The fluid conduits 130 may be hose pipes of different lengths and diameter. Segments of different dimension may be fluidically coupled using appropriate adaptors, or may be provided separately. The conduits may also be attached to the inside wall of the respective container(s) 102, 104, so that the first simulacrum 124 moves with the inflation of the containers 102, 104. In an example, the pipes 130 are flexible so as to suitably bend and flex with the breathing motion.

A third simulacrum 132 in the set may comprise one or more substantially solid blocks which are provided to mimic calcifications. For example, a Hounsfield unit for the third simulacrum may be greater than +120. The third simulacrum 132 may be provided within one or both of the first and second fluid containers 102, 104.

A fourth simulacrum 134 in the set of tissue simulacrum comprises one or more fluid bags comprising a third aqueous fluid. The fourth simulacrum 134 may be provided to mimic various organs found in the chest. For example, the fourth simulacrum may comprise fluid bags provided to mimic a heart and a liver. Suitably, the third aqueous fluid may be different to the first and second aqueous fluids, so that the fourth simulacrum appears differently on the medical imaging. By way of example, for CT scans a Hounsfield unit for the fourth simulacrum may be from about +35 to about +66, and for MR imaging a T1 value may be 900ms and a T2 value 50ms. It will be appreciated that the fourth simulacra 134 are disposed between the first or second fluid container 102, 104, and the ribs 116, so as to be suitable housed within the ribcage 110. In this way, the ribs 116 cause the fourth simulacra 134 to compress against the fluid containers 102, 104 during inflation, thereby creating appropriate artefacts in a medical image.

A fifth simulacrum 136 may also be used comprising one or more fluid bags comprising a fourth aqueous fluid. The fifth simulacrum 136 may be provided to mimic bodily tissues such as fat and muscle. Suitably, for CT scans a HU value of the fifth simulacrum may be in the range of about -70 to about -30, and for MR imaging a T1 value may be about 250ms and a T2 value about 70ms (for fat) or a T1 value about 900ms and a T2 value about 50ms (for muscle). The fifth simulacrum 136 may be disposed around the ribcage 110 so as to represent e.g., intercostal tissue. That is, the fifth simulacrum may be disposed in between separate ribs 116, in between the ribs 116 and a proximate fluid container 102, 104, or on an outer surface of the ribs 116 (i.e., the ribs 116 may be between the fifth simulacrum 136 and the fluid containers 102, 104).

It may also be desirable for the phantom 100 to mimic a quasi-realistic appearance for a visible light imaging device used as part of the medical imaging (e.g., sensor 204 in Fig. 1). Suitably, the phantom 100 may also comprise a material covering 138, such as clothing fabric. As an alternative to a material covering, the phantom may comprise a non-metallic skin-like mask 138.

To generate the inhale and exhale motions, the phantom comprises pumping means 140 which are hydraulically coupled to the first and second fluid containers 102, 104; for example, via the piping 106. The pumping means 140 is configured to sequentially inflate and deflate the pair of fluid containers 102, 104.

For example, the pumping means 140 may comprise a third flexible fluid container 142 (i.e., an inflatable bag) hydraulically coupled to the first and second flexible fluid containers 102, 104, and means to apply pressure to the third container 142. Such means may take the form of a pair of boards 144, 146 to which the third container is attached. One of the boards 144 may be fixed in place, while the other board 146 may be moveable towards and away from the fixed board 144. In this way, moving the moveable board 146 towards the fixed board 144 causes the third fluid container 142 to be squeezed between the two boards 144, 146, and in doing so forces fluid into the first and second fluid containers 102, 104 to inflate them. Conversely, moving the moveable board 146 away from the fixed board 144 causes expansion of the third fluid container 142 to create a pressure differential which causes the first and second fluid containers 102, 104 to deflate.

The pumping means 140 may comprise a coupling 148 configured to be coupled to driving means to provide inhalation and exhalation cycles mimicking human breathing. Here, the coupling 148 is provided on a rear side of the moveable board 146 (the front side being attached to the container 142).

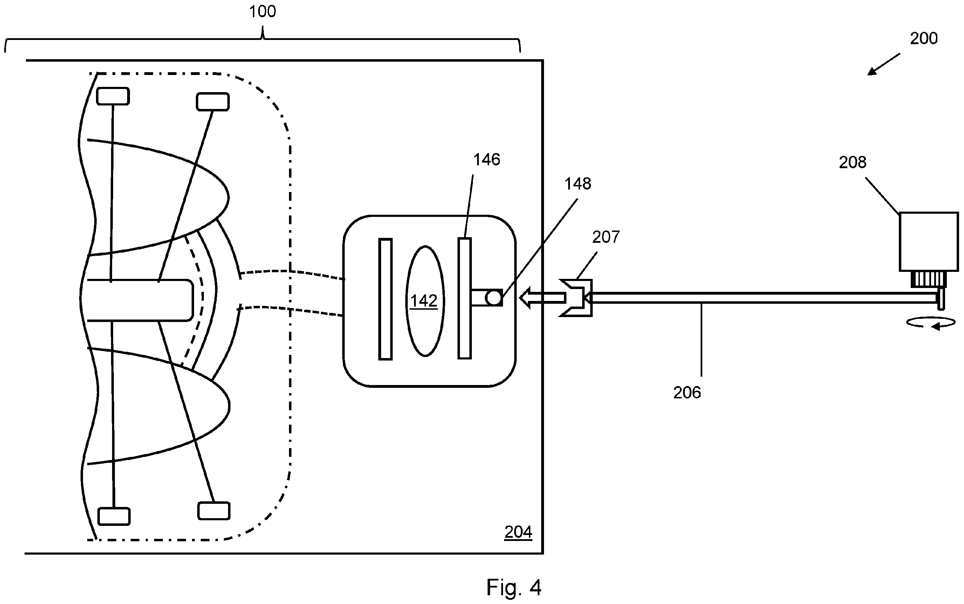

Fig. 4 shows an example apparatus 200 (or motion phantom) incorporating the chest phantom 100. Put another way, the apparatus 200 comprises a phantom object 202 to be imaged, which in this example is the chest phantom 100.

The apparatus 200 may comprise an imaging stage 204 configured to receive the phantom object. Here, the imaging stage 204 may comprise a table, or platform, onto which the phantom 100 is disposed. In an example, the entire phantom 100 may be disposed on the imaging stage/table 204, so that the imaging stage can be used for other purposes after use of the phantom 100.

In another example, the pumping means 140 of the phantom 100 may be fixedly attached to the imaging stage 204, or otherwise formed as an integral part of the imaging stage 204, such that the pumping means may be considered an element of the imaging stage 204. That is, the imaging stage 204 may comprise pumping means 140 for sequentially inflating and deflating the pair of fluid containers 102, 104. Suitably, in this example, mounting and demounting the phantom 100 from the imaging stage 204 comprises hydraulically coupling the pumping means 140 to the first and second fluid containers 102, 104: for example, by connecting the third fluid container 142 to the first and second containers 102, 104.

The apparatus comprises a linear actuator 206. The linear actuator 206 is provided as an elongate push bar, so that a means for driving the linear actuator 206 may be positioned far from the phantom 100 and thereby not interfere with any medical imaging.

The linear actuator is configured to couple to the coupling 148 of the pumping means. Suitably, the linear actuator 206 and coupling 148 may comprise mutually cooperating engagement means. For example, the coupling 148 may take the form of a male engagement part, while an end 207 of the actuator 206 may take the form of a female engagement part. In the example shown, the coupling 148 comprises a bar, or other protrusion, while the end 207 comprises a pair of grips configured to surround the bar/protrusion of the coupling 148. The grips may be inwardly biased so as to clip around the bar/protrusion of the coupling 148, or may be secured in position once engaged using other techniques as will be appreciated in the art.

Suitably, the coupling 148 is configured such that motion of the linear actuator 206 provides substantially linear push and pull motions to drive the pumping means 140: more specifically, to provide the pushing force to squeeze the third container 142 between the moveable board 146 and the fixed board 144 to cause the first and second containers 102, 104 to inflate, and the pulling motion to expand the third container 142 to cause the first and second containers 102, 104 to deflate.

While the linear actuator 206 could feasibly be driven by hand, or in general any suitable means, it is preferred to drive the linear actuator 206 (and therefore, in turn, the pumping means 140) by a controllable driver 208, such as a stepper (or other form of) motor. This allows for motion with controllable amplitude and speed and, hence, offers reproducibility.

In the context of Fig. 1, an imaging sensor 14 of an imaging system 10 may be suitably configured to capture a plurality of images synchronized with certain steps of the stepper motor 208, such that image capture is synchronized with the inflation and deflation of the fluid containers 102, 104 of the chest phantom 100 (e.g., at a mid-point of an inhale and an exhale).

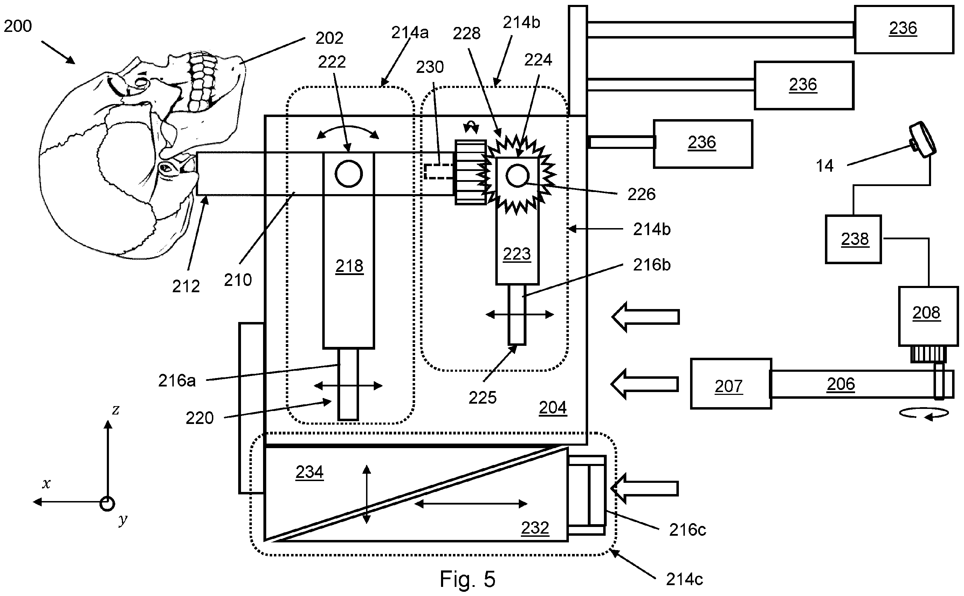

Fig. 5 shows another example apparatus 200' for calibrating (or otherwise testing on) a medical imaging system 10, optionally for use with a chest phantom 100, but preferably for use with other forms of phantoms. That is, the phantom object 202 may be a head (as illustrated), a limb, a hand, foot, and so on. The phantom object 202 may be formed from suitable materials (of e.g., suitable HU value and/or T1 and T2 values) to provide image content and image artefacts in the medical imaging. To adapt the phantom object 100 for visible image sensing by sensor 14, the phantom object may be provided with a non-metallic skin-like mask and/or optionally, clothing material, depending on the specific phantom object 202.

The imaging stage 204 configured to receive the phantom object 202 may be arranged to at least partly suspend the phantom object 202, so that the object 202 has freedom to move. In this example, the imaging stage 204 comprises a bar 210 configured to attach to the phantom object 202 at one end 212. The end 212 and object 202 may comprise mutually cooperating releasable engagement means, so that the phantom object 202 may be readily swapped (for example, a head for a hand, or for the chest phantom 100).

The imaging stage 204 comprises at least one coupling means 214 by which the phantom object 202 may be moved. The coupling means 214 are configured to generate at least one of a tilting motion (i.e., rotation back-to-front, for example in an *x, z* plane, analogous to nodding), a rotating motion (i.e., rotation left-right, for example in a *y, z* plane), and a lifting motion (i.e., translation up down, for example in a z axis). Put another way, the coupling means 210 may be at least one of a first coupling means 214a configured to tilt the phantom object 202, a second coupling means 214b configured to rotate the phantom object 202, and a third coupling means 214c configured to lift (and subsequently lower) the phantom object 202.

It will be appreciated that the stage 204 may comprise each of the first to third coupling means 214a-c as a set of coupling means. The set of coupling means 214 need not comprise all three coupling means, but rather may be formed from at least any two of the first to third coupling means 214a-c. For example, the set of coupling means may comprise at least the first coupling means 214a and the second coupling means 214b. Suitably, the set may be comprised of coupling means of different types, but in some embodiments the set of coupling means may comprise multiple means of the same type: e.g., two second (rotating) coupling means 214b, where a particularly complicated movement of a phantom object is desired. In the case of particularly heavy phantoms (as is likely to be the case for a head phantom), the imaging stage 204 may comprise one or more counterweights 236 configured to support the at least one tilting motion, rotating motion, and a lifting motion so that the power unit does not have to lift or otherwise move the full mass of the phantom.

The linear actuator 206 is configured to couple to the at least one coupling means 214, such that the linear actuator 206 drives the coupling means 214 to generate the appropriate sort of movement. Where a set of coupling means 214a-c are provided, in one example the linear actuator may be configured to couple to each of the coupling means 214a-c at the same time, so as to drive multiple types of motion simultaneously. In another example, the linear actuator 206 may be configured to drive a subset of the set of coupling means simultaneously (e.g., any two of the three couplings). In yet another example, the linear actuator 206 may be configured to drive only one of the set of couplings at a time, but may be suitably configured to be interchangeable between different coupling means (e.g., interchanged between at least the first coupling means 214a and second coupling means 214b).

Suitably, similar the coupling 148 of the, the linear actuator 206 may comprise an end 208 comprising parallel grips forming a female engagement part configured to engage with a male engagement part of the couplings 214 which may take the form of a bar, or rod, 216 which the grips of the linear actuator 206 cooperate with.

To generate a tilting motion, the first coupling 214a may comprise a member 218 arranged with a first end 220 coupled to the linear actuator 206 (e.g., via the rod 216a) and a second end 222 fixedly attached to the imaging stage, here to the bar 210, so that movement of the actuator 206 forwards and backwards (i.e., in the x direction) causes tilting of the phantom object.

To generate rotation, the second coupling 214b may comprise a member 223 having first end 225 coupled to the linear actuator 206 (e.g., via the rod 216b) and a second end 224 coupled to a first drive shaft 226 of a gear system 228. The gear system 228 comprises a second drive shaft 230 arranged at an angle to the first drive shaft 226; the angle is 90 degrees in the present example. The gear system 228 may comprise a beveled gear arrangement or worm gear arrangement, so that linear motion of the actuator 206 in the x direction is converted, via the first drive shaft 226, to rotational motion in the *y, z* plane at the second drive shaft 230. The second drive shaft 230 may be functionally the same element as the bar 210, or may be attached to an end of the bar 210 opposite to the end 212 coupled to the phantom object 202; for example by being inserted into the bar 210.

To generate lifting, the third coupling means 214c may comprise a pair of abutting wedges 232, 234. The first wedge 232 in the pair may be coupled to the linear actuator 206 (via the rod 216c) and the second wedge 234 in the pair fixedly attached to the imaging stage 204. A pushing movement of the linear actuator 206 in the positive x direction thereby causes the first wedge 232 to push against the second wedge 234 which in turn drives the imaging stage upwards in the positive z direction. Conversely, a pulling movement of the linear actuator 206 in the negative x direction withdraws the first wedge 232 from the second wedge 234, causing the imaging stage 204 (with second wedge 234) to fall under gravity in the negative z direction. In other words, pushing and pulling the first wedge 232 using the linear actuator causes the second wedge 234 / imaging stage 204 to rise and fall.

As with Fig. 4, the apparatus 200 of Fig. 5 may comprise a power unit 208, such as a stepper motor, configured to drive the linear motion of the actuator 206. Likewise, an imaging sensor 14 of a medical imaging device 10 may be configured to capture images on the phantom object 202 undergoing motion. Suitably, the apparatus 200 may comprise a controller 238 configured to transmit a trigger signal to the image sensor 14 to capture an image based on the operation of the motor 208. Put another way, the controller 238 may be interfaced to the medical imaging system 10 so that the power unit 208 and the imaging sensor 14 are operated in a fixed time relation to an image acquisition.

In summary, exemplary embodiments of improved motion phantoms have been described. The described exemplary embodiments are convenient to manufacture and straightforward to use. The exemplary embodiments may be manufactured industrially. An industrial application of the example embodiments will be clear from the discussion herein.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measured cannot be used to advantage. Any reference signs in the claims should not be construed as limiting the scope.

## Claims

1. An apparatus (200) for a medical imaging system (10), comprising:
a phantom object (202) to be imaged;
a linear actuator (206);
an imaging stage (204) configured to receive the phantom object; and
coupling means (214) configured to couple the imaging stage to the linear actuator;
wherein the coupling means are configured to generate at least one of a tilting motion (214a), a rotating motion (214b), and a lifting motion (214c), of the phantom object, when the linear actuator is in operation.

2. The apparatus of claim 1, wherein the coupling means is a first coupling means in a set of coupling means, and the set comprises a second coupling means configured to generate at least one of a tilting motion, a rotating motion, and a lifting motion, of the phantom object, different to the first coupling means.

3. The apparatus of claim 2, wherein the linear actuator is configured to be interchangeable between the first coupling means and second coupling means.

4. The apparatus of any preceding claim, wherein the coupling means configured to generate titling motion comprises a member (218) with a first end (220) coupled to the linear actuator and a second end (222) fixedly attached to the imaging stage.

5. The apparatus of any preceding claim, wherein the coupling means configured to generate rotating motion comprises a member (223) with a first end (225) coupled to the linear actuator and a second end (224) coupled to a first drive shaft (226) of a gear system (228), the gear system comprising a second draft shaft (230) oriented at an angle to the first drive shaft.

6. The apparatus of claim 5, wherein the gear system comprises a beveled gear system or a worm gear system.

7. The apparatus of any preceding claim, wherein the coupling means configured to generate lifting motion comprises a pair of abutting wedges having a first wedge (232) coupled to the linear actuator and a second wedge (234) coupled to the imaging stage, and wherein pushing and pulling the first wedge using the linear actuator causes the second wedge to rise and fall, respectively.

8. The apparatus of any preceding claim, further comprising a power unit (208) configured to drive the linear actuator.

9. The apparatus of any preceding claim, further comprising an imaging sensor (14) arranged to capture images of the phantom object in motion.

10. The apparatus of claims 8 and 9, further comprising a controller (238) configured to transmit a trigger signal to the image sensor to capture an image based on the operation of the power unit.

11. The apparatus of any preceding claim, wherein the phantom object is a head phantom.

12. The apparatus of claim 11, wherein the imaging stage comprises one or more counterweights (236) configured to support the at least one tilting motion, rotating motion, and a lifting motion.

13. The apparatus of any of claims 1 to 10, wherein the phantom object is a chest phantom.

14. The apparatus of any of claims 1 to 10, wherein the phantom object is a hand phantom.

15. The apparatus of any preceding claim, wherein the phantom object comprises a non-metallic skin-like mask.
